# EUROPEAN PATENT APPLICATION

(11) **EP 3 653 126 A1**
(43) Date of publication of application: **20.05.2020**
(21) Application number: 18831780.4
(22) Date of filing: 03.07.2018
(51) Int. Cl.: A61B 8/00, A61F 5/01

(54) **METHOD FOR ADJUSTING ORTHOSIS**

(30) Priority: 11.07.2017 CN 201710563308
(71) Applicant: Telefield Medical Imaging Limited, Hong Kong (CN)
(72) Inventor: ZHENG, Yongping, Hong Kong (HK); MAK, Takman, Hong Kong (HK)
(74) Representative: Hellmich, Wolfgang
(86) International application number: PCT/CN2018/094307
(87) International publication number: WO 2019/011157

(57) **Abstract**

A method for adjusting orthosis (200), comprising following steps: S1. by means of a detection window (101) on the orthosis, obtaining, by an ultrasound probe, a series of two-dimensional images and corresponding spatial position and angle information of human body-related parts needing to be corrected; S2. performing image reconstruction on the basis of the obtained series of two-dimensional images having spatial positioning information to obtain three-dimensional images and images on sections; S3. determining, according to information about skeletons on the two-dimensional or three-dimensional images, whether the adjustment provided by the orthosis (200) is correct; S4. adjusting the orthosis (200) according to the result in step S3. According to the method, the three-dimensional imaging of a skeletal system can be implemented without radiation, so that the evaluation of the correction result can be facilitated, and the orthosis (200) is adjusted to achieve the optimal adjustment or fixing effect.

## Description

### TECHNICAL FIELD

The present application relates to the field of medical devices, in particular to a method for adjusting an orthosis.

### BACKGROUND

In the medical field, it is often necessary to correct or fix the skeleton. For example, for patients of scoliosis with curvature more than a certain degree, orthosis should be given to improve the scoliotic curve or stabilize the therapeutic effect of scoliosis; when fracture, the skeleton in the corresponding part must be fixed. However, in the process of correction or fixation, it is usually not easy to judge whether the correction or fixation is correct with the naked eye from the outside of the human body, especially the concerned parts are often covered by the orthosis, so it is more difficult to judge the results of correction or fixation with the naked eye without removing the orthosis.

At present, the method used in the medical field is to know the state of the skeleton to be adjusted in the orthosis through X-ray. But X-ray imaging has radiation hazards, and X-ray imaging can only provide a single direction of projection image, and cannot provide three-dimensional information of skeletons. For example, in scoliosis orthotics, it is possible that the angle of one direction is reduced by an orthosis, but it cannot guarantee that the deformity or rotation of the spine in other directions is increased. Therefore, X-ray imaging is often needed in several directions. Thus, the amount of radiation is added, which in turn increases the potential harm to the human body.

Therefore, how to get the three-dimensional information of skeleton without removing the orthosis to accurately observe and evaluate the correction results, so that the orthosis can be adjusted to further strengthen or stabilize the orthotic effect, has become a technical problem that needs to be solved in the industry.

### SUMMARY

The purpose of the present application is to provide a method for adjusting an orthosis aiming at the existing technical problems, so that the three-dimensional imaging of a skeletal system can be implemented when wearing the orthosis and when there is no radiation, so that the evaluation of the correction result can be facilitated, and the orthosis is adjusted to achieve the optimal adjustment or fixing effect.

The technical scheme of the present application for solving the above technical problems is as follows, providing a method for adjusting an orthosis, wherein, comprising the following steps:
S1. by means of a detection device, using ultrasound probe to scan parts to be detected, to obtain a series of two-dimensional ultrasound images having spatial positioning information;
S2. performing image reconstruction on the basis of the obtained series of two-dimensional ultrasound images having spatial positioning information to obtain three-dimensional images and two-dimensional images on required sections;
S3. determining, according to information of parts to be detected displayed on the three-dimensional images and the two-dimensional images on the required sections, whether a correction result provided by the orthosis is correct;
S4. adjusting the orthosis according to the correction result.

Preferably, when the orthosis fails to reach the required correction result, the adjusting method further comprises:
S1-1. using ultrasound probe to scan parts to be detected that are not covered by the orthosis, to obtain a series of two-dimensional ultrasound images having spatial positioning information.

Preferably, before the step S1 the adjusting method further comprises the following steps:
S0. when detection body not wearing the orthosis, scanning the detection body to obtain information of the whole skeleton before the orthosis;
or,
after the step S4 further comprising the following steps:
   S5. repeating the above steps S1 to S4 until the orthosis achieves the required correction result.

Preferably, the step S1 further comprises:
S1-2: applying a plurality of ultrasound probes, by repeatedly scanning through a plurality of the detection devices, to obtain and display the three-dimensional images and the two-dimensional images on the required sections through real-time imaging.

Preferably, the ultrasound probe is a three-dimensional ultrasonic imaging probe.

Preferably, the adjustment in step S4 is performed continuously in the real-time imaging process.

Preferably, the detection device is a detection window, with the size of the detection window allows the ultrasound probe to directly contact the parts to be detected to detect the parts to be detected.

Preferably, the orthosis comprises a reinforcing device arranged on the orthosis, used for reinforcing the detection device.

Preferably, the reinforcing device comprises a reinforcing side or a reinforcing frame, arranged on edge of the detection device, used for reinforcing the detection device; a reinforcing plate, movably connected with the reinforcing side or the reinforcing frame, for closing and opening for the detection device.

Preferably, the detection device comprises a detection window, on the orthosis or outside of the orthosis, which is an opening for allowing the ultrasound probe to extend inside of the orthosis to detect the parts to be detected; or a detection area which is an area on the orthosis made of a material easy for ultrasound to penetrate.

Preferably, when the three-dimensional images and the two-dimensional images of the required sections are discontinuous local images of the skeleton, the adjusting method further comprises the following steps:
S6. estimating, through the three-dimensional images and the two-dimensional images of the required sections, information of undetected parts of the skeleton to form overall information of the skeleton.

Preferably, setting of the detection device on the orthosis is based on strength requirements of the orthosis and requirement that the three-dimensional images and two-dimensional images on the required sections can cover information of the parts to be detected as much as possible.

Preferably, the spatial positioning information is obtained according to the relative position and angle of the ultrasound probe on the orthosis.

Preferably, the spatial positioning information is obtained by a three-dimensional spatial positioning device installed on the orthosis, including a transmitter and/or a receiver of the three-dimensional spatial positioning device installed on the orthosis.

By applying the method for adjusting an orthosis of the present application, the three-dimensional imaging of a skeletal system and the two-dimensional images information of the required sections can be implemented when the detection body wearing the orthosis without radiation, so that the evaluation of the correction result can be facilitated, and the operator can adjust the orthosis in real time to achieve the optimal adjustment or fixing effect.

### BRIEF DESCRIPTION OF THE DRAWINGS

The application will be further described in combination with the accompanying drawings and embodiments, in which:
Figure 1 is the flow chart of the method for adjusting an orthosis of the present application;
Figure 2 is the structure diagram of the orthosis in a preferred embodiment of the present application;
Figure 3 is the structure diagram of the detection device in the first embodiment of the present application;
Figure 4 is the structure diagram of the detection device in the second embodiment of the present application;
Figure 5 is the diagram of the three-dimensional images obtained in a preferred embodiment of the present application;
Figure 6 is the structure diagram of the detection device in the third embodiment of the present application.

### DETAILED DESCRIPTION OF THE PREFERRED EMBODIMENT

In order to enable those skilled in the art to understand the application more clearly, the application will be described in further detail below in combination with the drawings and specific embodiments.

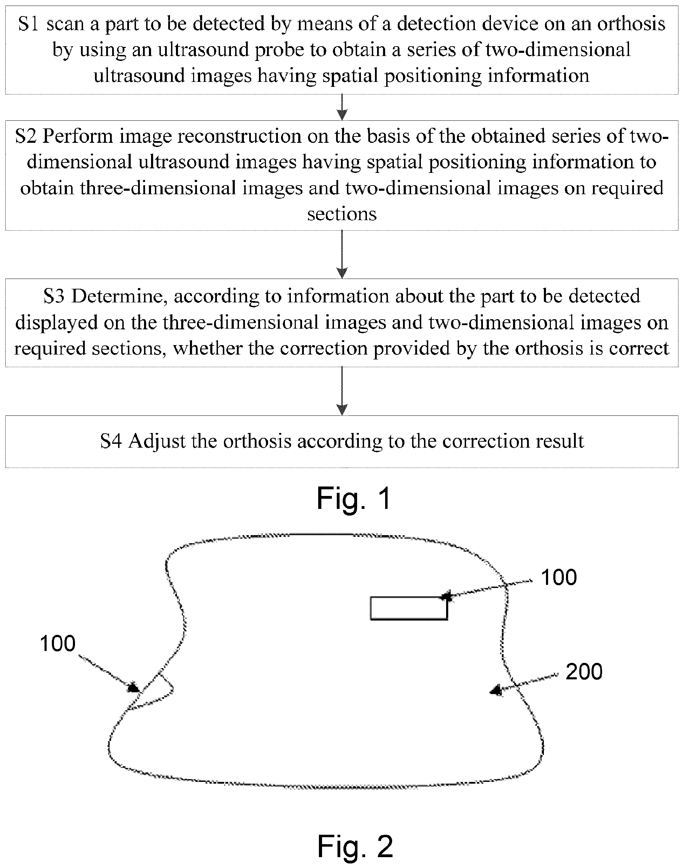

As shown in Figure 1, the present application discloses a method for adjusting an orthosis. As shown in Figure 2, the orthosis in the adjusting method comprises all devices and functions of the traditional orthosis 200, and the detection device 100. The method for adjusting an orthosis provided by the present application comprises the following steps:
by means of a detection device 100, using ultrasound probe to scan parts to be detected, to obtain a series of two-dimensional ultrasound images having spatial positioning information (step S1). The ultrasound probe is a 3D ultrasound probe or an ultrasound probe of a 3D ultrasonic imaging system, and the detection device 100 can be one or more. In the step S1, if the two-dimensional ultrasound image obtained in one scan is not enough to provide the display of the whole parts to be detected, step S1 can be repeated until the two-dimensional ultrasound image obtained can display the whole parts to be detected, and the scanning is completed. The spatial positioning information includes spatial position information and angle information. The spatial positioning information can be obtained by installing a spatial positioning sensor on the ultrasound probe to obtain the spatial position information and angle information of the corresponding ultrasound probe of each two-dimensional ultrasound image, so as to obtain the spatial positioning information of each two-dimensional ultrasound image. Preferably, the ultrasound probe is provided with a micro mechanical scanning device, so that the ultrasound probe can automatically complete omni-directional scanning through the detection device 100. It can be understood that step S1 further includes adding enough ultrasound couplant or coupling block in the detection device 100 or on the detection device 100, so as to facilitate the scanning of the ultrasound probe (S1-3). At the same time, the step S1 further includes: S1-1. using ultrasound probe to scan parts to be detected that are not covered by the orthosis, to obtain a series of two-dimensional ultrasound images having spatial positioning information.

Wherein, the spatial positioning information is obtained according to the relative position and angle of the ultrasound probe on the orthosis. The shape of the orthosis is basically fixed after the design, and the relative position of each detection device 100 on the orthosis is also fixed, so as long as the rotation of the ultrasound probe at the detection device 100 is obtained, the spatial position and angle of each two-dimensional ultrasound image can be obtained accordingly, without the need for additional three-dimensional spatial positioning device. In addition, the spatial positioning information can also be obtained by a three-dimensional spatial positioning device installed on the orthosis, including a transmitter and / or a receiver installed on the orthosis.

Performing image reconstruction on the basis of the obtained series of two-dimensional ultrasound images having spatial positioning information to obtain three-dimensional images and two-dimensional images on required sections (step S2).

Determining, according to information of parts to be detected displayed on the three-dimensional images and the two-dimensional images on the required sections, whether a correction result provided by the orthosis is correct (step S3). The correction result refers to that, when the orthosis is adjusted, the orthosis acts on the human bone and corrects or stabilizes the bone. The correction results can be adjusted in real time by adjusting the orthosis in real time.

Adjusting the orthosis according to the correction result (step S4). The adjustment of orthosis can be either overall correction or local adjustment, and the local adjustment can be shape adjustment or adjustment with adjustment pad.

If steps S1 to S4 are not enough to achieve the best correction result, repeat steps S1 to S4 until the required correction result is achieved for the orthosis (step S5).

Understandably, during the adjustment process, that is, in the process of performing steps S2 to S4, applying a plurality of ultrasound probes, by repeatedly scanning through a plurality of the detection devices 100, to obtain and display the three-dimensional images and the two-dimensional images on the required sections through real-time imaging, for example, related two-dimensional skeleton map, letting the adjuster get the feedback of correction in time, so as to make continuous and more effective adjustment. The repeated scanning can be automatically completed by the ultrasound probe with mechanical scanning function. Preferably, a plurality of ultrasound probes with mechanical scanning function are used to scan through different detection devices 100 at the same time, so that a large range of correction conditions can be obtained in real time. Because the change of skeleton inside the orthosis will lead to the change of skeleton in other parts, in order to get better correction, the scope of scanning is not limited to the human body part inside the orthosis, but also the part outside the orthosis can be imaged by the above methods to obtain the whole skeleton or skeleton structure for the reference of the adjuster.

Preferably, the ultrasound probe is a three-dimensional ultrasound imaging probe, which can provide positioning reference by mechanical scanning one-dimensional ultrasound transducer array or electronic scanning two-dimensional ultrasound transducer array. The ultrasound probe can also be flat, which is convenient to extend into the orthosis.

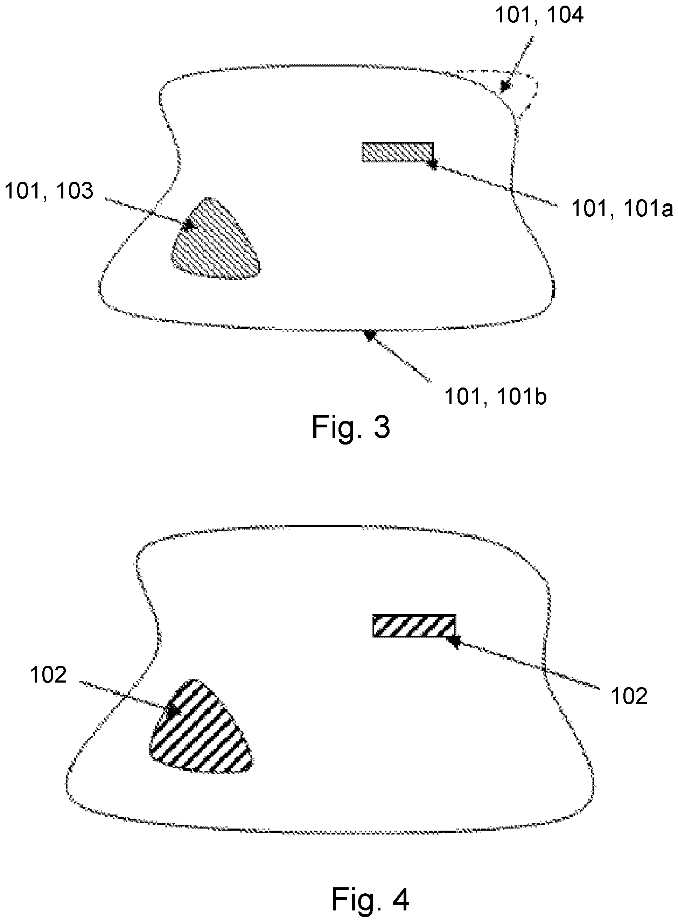

In order to make the above method more convenient and immediately applicable to the existing orthosis, the detection device 100 can be a detection window 101 (as shown in Fig. 3) or a detection area 102 (as shown in Fig. 4); the difference between the two is that the size of the detection window 101 allows the ultrasound probe to extend to the opening inside the orthosis for detection of the part to be detected, which can be located on the orthosis, as shown in the first detection window 101A; it can also be located outside the orthosis, as shown in the second detection window 101b; the first detection window 101a and the second detection window 101b are both detection windows 101, while the detection area 102 is the area on the orthosis made of a material for ultrasound to easily penetrate.

As shown in Fig. 3, for the existing orthosis or the orthosis which are not designed based on the method of the application, generally there will be no detection area, but they generally have a certain number of openings 103, vacancies 104 or hollow positions. Therefore, the openings 103 on the existing orthosis or outside the orthosis can be directly used as the detection window 101, and the orthosis can also be detected by the method of the application. In the orthosis specially designed based on the method of the application, the detection window 101 can be directly designed at the stage of orthosis design, and the detection device 100 can be designed as the detection window 101 or the detection area 102, which is not limited here.

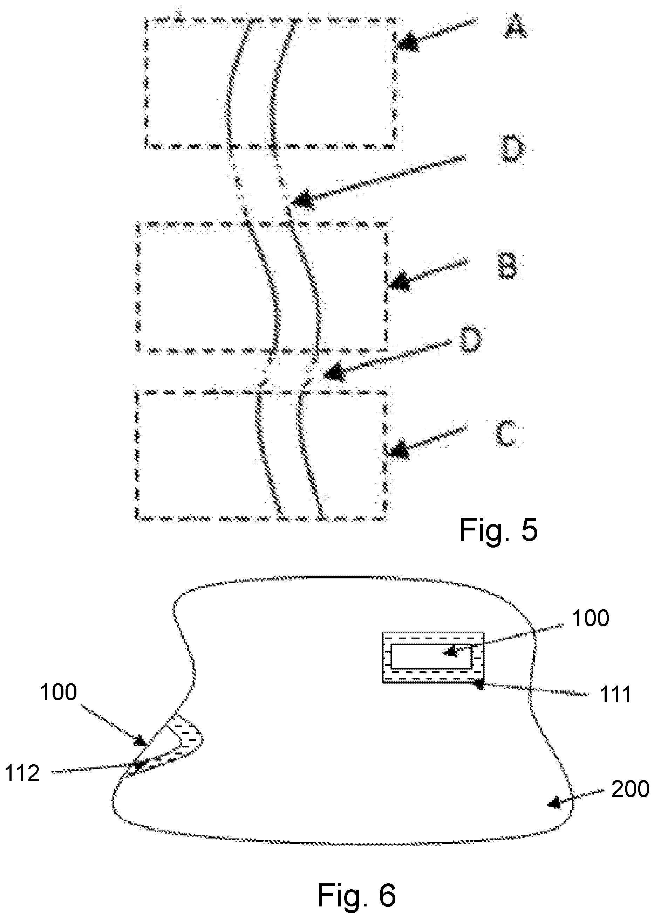

As shown in Fig. 5, since the three-dimensional image and the two-dimensional image are limited by the size and direction of the detection device 100, the obtained three-dimensional image and the two-dimensional image do not necessarily fill the entire three-dimensional space, that is, the obtained three-dimensional image and the two-dimensional image are discontinuous local images of the skeleton to be detected. However, since the skeleton, such as the spine, is an overall structure, the information of the undetected skeleton part can be estimated from the local image to form the overall skeleton information (step S6), so as to infer the overall deformation. In the figure, the first area A, the second area B and the third area C are imaged by ultrasound probe scanning, while the undetected skeleton part D is obtained by estimation. The estimation can be carried out by computer algorithm, or the human standard bone structure can be stored in the image processing system. By fitting the imaged area with the standard bone structure, the skeleton morphology of the undetected part can be simulated.

In order to obtain the correction of the skeleton with as few detection devices 100 as possible, the method also includes scanning the detection body when the orthosis is not worn, so as to obtain the whole skeleton condition (S0) before the adjustment, such as the three-dimensional imaging of the spine and different sections. Then wear the upper orthosis, and get the local skeleton changes after adjustment through steps S1-S4. Using these local information, we can adjust the 3D image, 2D section or 3D skeleton detected when we don't wear orthosis, so it is easier to get the whole skeleton state after adjustment with limited local information.

As shown in Fig. 2, the orthosis in the adjustment method includes all devices and functions of the traditional orthosis 200, as well as the detection device 100. The ultrasound probe scans the human body through the detection device 100 to obtain the three-dimensional image of the skeleton. The following is an embodiment of the detection device 100 involved in the adjustment method.

### The first embodiment

As shown in Fig. 3, the detection device 100 is one or more detection windows 101, and the size of the detection windows 101 can allow the ultrasound probe to extend to the interior of the orthosis to scan the part to be detected. Preferably, the ultrasound probe can rotate 365 degrees inside the orthosis. The detection window 101 can be located on the orthosis, for the opening 103 or the vacancy 104 on the orthosis, or outside the orthosis, as long as it can let the ultrasound probe enter into the interior of the orthosis for detection, there is no limit here.

If a detection window 101 is not enough to cover all the parts to be detected, multiple detection windows 101 with different directions and sizes can be set at different positions of the orthosis. The premise of setting the above detection window 101 is that the function, strength and comfort of the orthosis cannot be affected, so the selection of the position, size and direction of the detection window 101 needs to be considered at the beginning of the orthosis design, and carefully calculated and selected. On the one hand, to achieve the purpose of scanning with the ultrasound probe, on the other hand, not to affect the function of orthosis, the setting of the detection window 101 on the orthopedic brace is based on the strength requirements of the orthosis, and the three-dimensional image and the two-dimensional image on the required section can cover the position information to be detected as much as possible. Therefore, the manufacturing process of the orthosis can also include computer simulation calculation. Through the shape of the orthosis, the mechanical properties of the orthosis material and the force and direction of the orthosis after wearing to the corresponding parts of the human body, the position of the detection window 101 and the size and direction of the detection window 101 can be calculated and designed. At the same time, it can also predict the parts to be scanned through the detection window 101 through simulation calculation. That is, the manufacturing method of the orthosis comprises at least the following steps: 1, the model for the orthopaedic part is extracted as the first model; 2, the orthosis are used for orthopedic treatment of the parts to be detected; when the correction results reach the best, the model of the temporary orthosis is extracted as the second model; 3, the first model and the second model are analyzed by the processor, and the best position and direction of the detection device 100 are determined to establish orthodontic bracing models for orthotic parts; 4. The orthosis is produced by 3D printing, injection molding and other methods. It is understandable that when adjusting the temporary orthosis to obtain the second model, the correction result can be detected by the adjustment method of the application to obtain the second model with the best correction result. In the process of adjustment, we can also adjust the position relationship between orthosis and human body by adding cushion blocks between orthosis and human body.

Preferably, the ultrasound probe can be a flat probe, so that it can easily extend into the detection window 101 and scan the part to be detected. More preferably, the ultrasound probe has a micro mechanical scanning device, so that the probe can enter a window and complete automatic scanning. In this embodiment, enough ultrasound couplant or coupling block can be added directly on the part to be detected, so as to facilitate the scanning of ultrasound probe.

### The second embodiment

As shown in Fig. 4, the second embodiment of the application is different from the first embodiment in that the detection device 100 is a detection region 102. The detection area 102 is made of material for ultrasound to easily penetrate, which is used to move the ultrasound probe on the detection area 102 to detect the parts to be detected, instead of extending the ultrasound probe into the detection window in the first embodiment, that is, the interior of the orthosis can detect the parts to be detected. Preferably, the material of the whole orthosis is the same as the ultrasonic penetration material of the detection area 102, so that the ultrasound probe can carry out ultrasound detection at any position of the orthosis. In this embodiment, enough ultrasound couplant or coupling block can be added to the detection area 102 to facilitate the scanning of the ultrasound probe.

The embodiment of the application is presented in a progressive manner, only the differences between the present embodiment and the first embodiment are described, and the parts not described in the second embodiment are the same as the first embodiment

### The third embodiment

As shown in Fig. 6, the orthosis in the third embodiment of the present application further comprises a reinforcing device 110 for temporarily or permanently reinforcing the detection device 100. In this embodiment, the reinforcing device 110 is a reinforcing frame 111 or a reinforcing side 112. In this embodiment, the reinforcing device 110 is made of metal materials, which can be made of different types of metals, such as light aluminum, titanium and other metal materials, or special steel and other materials that are not easy to cause user allergy in contact with skin. For the detection device 100 located in different parts to be detected, different materials can be used. Preferably, the reinforcing device 110 also includes a reinforcing plate which can be movably connected with the reinforcing frame 111 or the reinforcing side 112 for opening the detection device 100 when it is necessary to use the detection device 100, or for temporarily or permanently closing the detection device 100 after the orthotic support is adjusted. The reinforcing plate can be made of metal material or soft material, such as silica gel. It can be understood that the detection device 100 in this embodiment can be a detection window or a detection area.

It can be understood that the above three embodiments can be implemented in one orthosis at the same time, that is, in the same orthosis, there are both detection windows and detection areas, as well as reinforcement devices for strengthening detection windows and detection areas. They can be designed and configured according to actual needs, without limitation here.

In summary, by using the method for adjusting the orthosis disclosed by the present application, the operator can quickly and intuitively obtain the orthopedic condition of the orthosis, and can also obtain the three-dimensional imaging of the skeleton system without any radiation. Thus, the orthotic efffects of the orthosis can be obtained in real time, and the orthosis can be effectively adjusted in real time when the orthosis is worn, so that the orthosis can achieve the best adjustment or fixation effect. The most important thing is that in the process of obtaining orthotic effects and adjusting the orthosis, it will not cause any radiation to the patient and avoid potential harm to the patient.

It should be understood that for a person of ordinary skill in the art, improvements or changes can be made according to the above description, and all these improvements and changes should fall within the protection scope of the appended claims of the present application.

## Claims

1. A method for adjusting an orthosis, wherein, comprising the following steps:
S1. by means of a detection device (100), using ultrasound probe to scan parts to be detected, to obtain a series of two-dimensional ultrasound images having spatial positioning information;
S2. performing image reconstruction on the basis of the obtained series of two-dimensional ultrasound images having spatial positioning information to obtain three-dimensional images and two-dimensional images on required sections;
S3. determining, according to information of parts to be detected displayed on the three-dimensional images and the two-dimensional images on the required sections, whether a correction result provided by the orthosis is correct;
S4. adjusting the orthosis according to the correction result.

2. The method for adjusting an orthosis according to claim 1, wherein, the step S1 further comprises:
S1-1. using ultrasound probe to scan parts to be detected that are not covered by the orthosis, to obtain a series of two-dimensional ultrasound images having spatial positioning information.

3. The method for adjusting an orthosis according to claim 1, wherein, before the step S1 the adjusting method further comprises the following steps:
S0. when detection body not wearing the orthosis, scanning the detection body to obtain information of the whole skeleton before the orthosis;
or,
after the step S4 further comprising the following steps:
S5. repeating the above steps S1 to S4 until the orthosis achieves the required correction result.

4. The method for adjusting an orthosis according to claim 1, wherein, the step S1 further comprises:
S1-2: applying a plurality of ultrasound probes, by repeatedly scanning through a plurality of the detection devices (100), to obtain and display the three-dimensional images and the two-dimensional images on the required sections through real-time imaging.

5. The method for adjusting an orthosis according to claim 1, wherein, the ultrasound probe is a three-dimensional ultrasonic imaging probe.

6. The method for adjusting an orthosis according to claim 4, wherein, the adjustment in step S4 is performed continuously in the real-time imaging process.

7. The method for adjusting an orthosis according to claim 1, wherein, the detection device (100) is a detection window (101), with the size of the detection window (101) allows the ultrasound probe to directly contact the parts to be detected to detect the parts to be detected.

8. The method for adjusting an orthosis according to claim 1, wherein, the orthosis comprises a reinforcing device (110) arranged on the orthosis, used for reinforcing the detection device (100).

9. The method for adjusting an orthosis according to claim 8, wherein, the reinforcing device (110) comprises:
a reinforcing side (112) or a reinforcing frame (111), arranged on edge of the detection device (100), used for reinforcing the detection device (100);
a reinforcing plate (113), movably connected with the reinforcing side (112) or the reinforcing frame (111), for closing and opening the detection device (100).

10. The method for adjusting an orthosis according to claim 1, wherein, the detection device (100) comprises:
a detection window (101), on the orthosis or outside of the orthosis, which is an opening for allowing the ultrasound probe to extend inside of the orthosis to detect the parts to be detected; or
a detection area (102) which is an area on the orthosis made of a material for ultrasound to easily penetrate.

11. The method for adjusting an orthosis according to claim 1, wherein, when the three-dimensional images and the two-dimensional images of the required sections are discontinuous local images of the skeleton, the adjusting method further comprises the following steps:
S6. estimating, through the three-dimensional images and the two-dimensional images of the required sections, information of undetected parts of the skeleton to form overall information of the skeleton.

12. The method for adjusting an orthosis according to claim 1, wherein, setting of the detection device (100) on the orthosis is based on strength requirements of the orthosis and requirement that the three-dimensional images and two-dimensional images on the required sections can cover information of the parts to be detected as much as possible.

13. The method for adjusting an orthosis according to claim 1, wherein, the spatial positioning information is obtained according to the relative position and angle of the ultrasound probe on the orthosis.

14. The method for adjusting an orthosis according to claim 1, wherein, the spatial positioning information is obtained by a three-dimensional spatial positioning device installed on the orthosis, including a transmitter and/or a receiver of the three-dimensional spatial positioning device installed on the orthosis.
